Europäisches Patentamt

European Patent Office

Office européen des brevets

(19)

(11) Veröffentlichungsnummer: **0 144 610**
**B1**

(12) # EUROPÄISCHE PATENTSCHRIFT

(45) Veröffentlichungstag der Patentschrift:
09.08.89

(21) Anmeldenummer: 84111982.9

(22) Anmeldetag: 05.10.84

(51) Int. Cl.⁴: **A 61 N 1/42**, A 61 N 1/10

(54) Galvanisches Element.

(30) Priorität: 17.11.83 DE 3341620

(43) Veröffentlichungstag der Anmeldung:
19.06.85 Patentblatt 85/25

(45) Bekanntmachung des Hinweises auf die Patenterteilung: 09.08.89 Patentblatt 89/32

(84) Benannte Vertragsstaaten:
AT BE CH FR GB IT LI LU NL SE

(56) Entgegenhaltungen:
EP–A– 0 036 022
DE–A– 2 648 232
DE–A– 3 106 060
DE–U– 6 904 160
DE–U– 8 128 911
FR–A– 2 284 341
FR–A– 2 336 949
FR–A– 2 422 409

(73) Patentinhaber: **Bohn, Gerhard**
**Wiesenstrasse 18**
**D-6433 Philippsthal (DE)**

(72) Erfinder: **Bohn, Gerhard**
**Wiesenstrasse 18**
**D-6433 Philippsthal (DE)**

(74) Vertreter: **Geyer, Ulrich F., Dr. Dipl.-Phys.**
**WAGNER & GEYER Patentanwälte Gewuerzmuehls-**
**trasse 5 Postfach 246**
**D-8000 München 22 (DE)**

EP 0 144 610 B1

Anmerkung: Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

**Beschreibung**

Die Erfindung betrifft ein galvanisches Element, in dem magnetisiebare Teilchen enthalten sind.

Magnetische Felder wurden zu Heilzwecken bereits im Altertum und im Mittelalter eingesetzt (vgl. z. B. W. Waldmann, Deutsches Arch. f. Geschichte d. Med. u. med. Geographie, Leipzig, 1878, S. 320 ff.). Insbesondere in diesem Jahrhundert wurde die therapeutische Wirkung von Magnetfeldern intensiv untersucht und deren Bedeutung für die Medizin festgestellt.

Es ist auch seit langem bekannt, dynamische und statische elektrische Felder in der Medizin zu verwenden.

Die Bedeutung von magnetischen und elektrischen Feldern in der Medizin wurde in den letzten Jahrzehnten nicht mehr in Frage gestellt, was durch Anerkennung der Heilwirkung durch diese Verfahren von der kassenärztlichen Bundesvereinigung unterstrichen wurde.

Aus dem Dokument DE 6 904 160 ist eine Vorrichtung zur Erzeugung eines magnetischen Feldes bekannt, bei der der Magnetträger ein folienförmiger, magnetisierbarer flacher Körper ist. Die magnetisierbaren flachen Körper oder Schichten sind dabei axial magnetisiert. Der Magnetkörper ist durch mehrere Metallschichten abgedeckt und durch Isolierschichten voneinander und von der Magnetschicht getrennt, wobei diese isolierten, miteinander verbundenen Metallschichten sinngemäß wie ein Flachkondensator wirken. Die Metallschichten dienen dabei der Bereitstellung eines elektrostatischen Felds.

Aus der FR-A-2 336 949 ist ein Verfahren und eine Anordnung bekannt, bei dem bzw. bei der in einem auf der Haut zu tragenden Textilteil ein Metalldrahtnetz von einem Elektrolyten umgeben ist, so daß ein elektrostatisches Feld im wesentlichen parallel zur Hauptoberfläche erzeugt wird. Über magnetische Felder ist in dieser Druckschrift nichts ausgesagt.

Der Erfindung liegt daher die Aufgabe zugrunde, ein galvanisches Element zu schaffen, mit dem auf einfache Weise die Anwendung magnetischer als auch elektrischer Felder auf Körper oder Körperteile des Menschen, beispielsweise im Schlafbereich, oder auf Tierkörper oder -körperteile mit ausreichend hohen magnetischen und/oder elektrischen Feldstärken ermöglicht wird.

Diese Aufgabe wird mit den im Anspruch 1 angegebenen Merkmalen gelöst.

Die Verwendung eines galvanischen Elements ergibt ein elektrostatisches Feld. Die magnetischen Teilchen sind dabei magnetisch so ausgerichtet, daß sich auf der einen Metallelektrode der magnetische Süd- und auf der anderen der magnetische Nordpol befindet. Bei der vorliegenden Erfindung werden magnetisierbare Teilchen zur Erzeugung des Magnetfelds verwendet.

Durch das galvanische Element ergibt sich ein elektrostatisches Feld und die magnetisierbaren Teilchen erzeugen ein magnetisches Feld, so daß mit dem erfindungsgemäßen galvanischen Element gleichzeitig die Applikation sowohl von elektrostatischen als auch magnetischen Feldern möglich ist. Durch die Überlagerung des elektostatischen und des magnetischen Felds ergibt sich nicht nur ein additiver, sondern darüberhinaus ein synergetischer Effekt, der über die einzelnen getrennten Anwendungsformen des elektrischen und des magnetischen Feldes hinaus in der Kombination eine besondere Wirkung zeigt. Das erfindungsgemäße galvanische Element ist insbesondere auch für eine langzeitige Applikation geeignet.

Gemäß einer bevorzugten Ausführungsform der Erfindung besteht das galvanische Element aus zwei Metallelektroden und einer zwischen diesen angeordneten Zwischenschicht, in der Graphit als Elektrolyt-Träger vorgesehen ist. Vorzugsweise besteht die Zwischenschicht aus einer Kautschukschicht bzw. einem auf Kautschukbasis beruhenden Material, in der bzw. in dem Graphit mit dem Elektrolyten eingelagert ist. Durch diese Ausführung ergibt sich ein sehr flexibles, in die unterschiedlichen Anwendungsfälle anpaßbares galvanisches Element.

In die Zwischenschicht sind magnetisierbare Teilchen, vorzugsweise Strontiumoxid oder Eisenoxid, eingelagert. Dieses Material ist zur Erzielung guter Magnetfelder besonders vorteilhaft. Insbesondere ist auch eine Mischung aus Strontiumoxid und Eisenoxid gut geeignet.

Gemäß einer weiteren Ausführungsform der Erfindung sind in der Zwischenschicht die Elektroden verbindende Bereiche vorgesehen, in denen unregelmäßig Graphit, das den Elektrolyten enthält, nicht jedoch magnetisierbare Teilchen eingelagert ist bzw. sind. Diese die Elektroden verbindenden Bereiche können stab- aber auch flächenförmig sein.

Vorzugsweise besteht die positive Elektrode aus Kupfer und die negative Elektrode aus Zink. Es ist jedoch auch möglich, als positive Elektrode Gold und als negative Elektrode Silber zu wählen. Je nach Anwendungsfall ist es jedoch auch möglich, andere Kombinationen chemischer Elemente zu verwenden, wobei jedoch darauf zu achten ist, daß die jeweils verwendeten chemischen Elemente einer Kombination in der Spannungsreihe möglichst weit auseinanderliegen.

Die Metallelektroden sind gemäß einer Ausführungsform auf die Zwischenschicht aufgedampft. Besonders vorteilhaft ist es jedoch, daß die Metallelektroden aus Metallpulver bestehen, das auf der jeweiligen Seite der Zwischenschicht, vorzugsweise einer Kautschukschicht, eingebracht ist. Bei starker Biegung des galvanischen Elements wird mit dieser Ausführungsform vermieden, daß die Elektroden brechen.

Das galvanische Element ist vorzugsweise in Form einer elastischen Folie ausgebildet. Dadurch ist es möglich, diese Folie allen Körperstellen anzupassen oder an Artikel, wie Kleidungsstücken, anzubringen, die nachgiebig und anpassungsfähig sind.

Eine besonders vorteilhafte Wirkung des galvanische Elements wird bei Langzeitanwendung festgestellt. Es ist daher insbesondere vorgesehen, derartige galvanische Elemente an Bettartikeln, wie Unterbetten, Oberbetten, Matratzenschonern usw., anzubringen, nicht zuletzt auch deshalb, weil festgestellt wurde, daß diese therapeutischen Effekte auch eine sehr positive Wirkung auf das Schlafverhalten ausüben.

Die Erfindung wird nachstehend anhand schematischer Zeichnungen beispielsweise näher erläutert. Es zeigen :

Fig. 1 eine schematische Querschnittsansicht gemäß einer Ausführungsform der Erfindung und

Fig. 2 eine schematische Querschnittsansicht gemäß einer weiteren Ausführungsform der Erfindung.

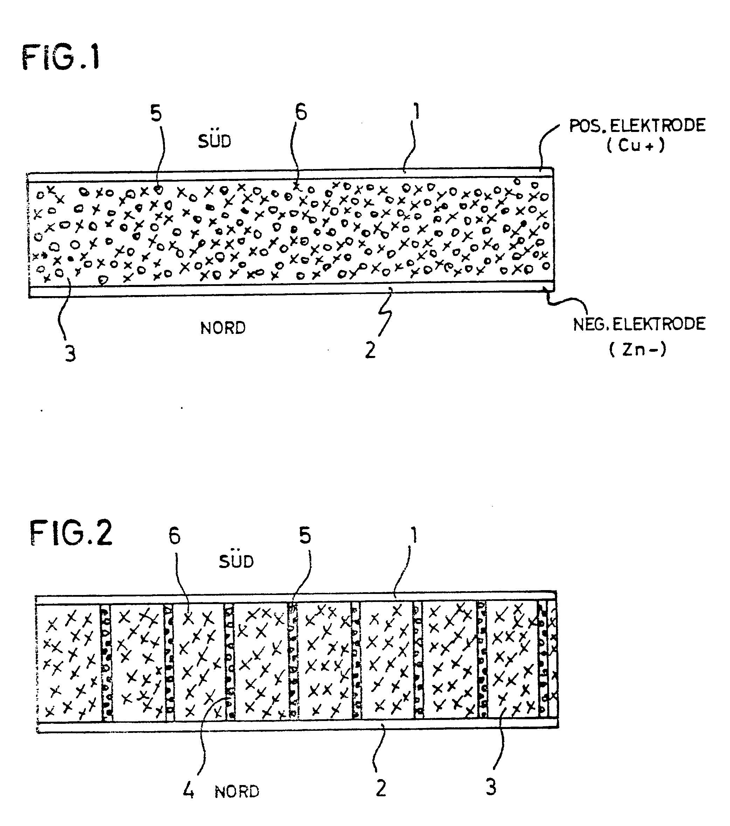

In Fig. 1 ist zwischen einer positiven Elektrode 1, die beispielsweise aus Kupfer bestehen kann, und einer negativen Elektrode 2, die beispielsweise aus Zink bestehen kann, eine Zwischenschicht 3 vorgesehen. Bei dieser Ausführungsform besteht diese Zwischenschicht aus einem auf Kautschukbasis bestehenden Material, in das als Elektolyt-Träger dienendes Graphit 5 — durch kleine Kreise angedeutet — und magnetisierbare Teilchen 6 — durch kleine Kreuze angedeutet — eingegeben ist. Die Metallelektroden sind auf die Kautschuk-Zwischenschicht vorzugsweise aufgedampft oder in einer anderen Weise aufgebracht. Die magnetisierbaren Teilchen 6 bestehen vorzugsweise aus Strontiumoxid (SrO) oder Eisenoxid ($Fe_2O_3$) oder aus einem Gemisch dieser Oxide bzw. aus $SrO \times 6 \, Fe_2O_3$. Die magnetisierbaren Teilchen sind bzw. werden magnetisch so ausgerichtet, daß sich auf der einen Seite der magnetische Süd- und auf der anderen Seite der magnetische Nordpol bildet.

In Fig. 2 wurden für die gleichen Teile dieselben Bezugszeichen verwendet. Zwischen den beiden Elektroden 1 und 2 befindet sich wiederum eine Zwischenschicht 3, in der jedoch die Elektroden verbindende, vorzugsweise senkrecht zu den Elektrodenflächen stehende Bereiche vorgesehen sind, in deren Graphit 5 als Elektrolytträger unregelmäßig eingelagert ist. Diese Bereiche dienen als Elektrolytbereiche und enthalten keine magnetisierbaren Teilchen. Die magnetisierbaren Teilchen 6, die wie im Ausführungsbeispiel gemäß Fig. 1 Strontiumoxid, Eisenoxid und/oder eine Mischung derselben bzw. anisotropes $SrO \times 6 \, Fe_2O_3$ sein können, befinden sich in den Bereichen der Zwischenschicht, die zwischen den Elektrolytbereichen liegen. Auch hier sind bzw. werden die magnetisierbaren Bestandteile magnetisch so ausgerichtet, daß sich auf der einen Seite dieser Folie der magnetische Süd- und auf der anderen der magnetische Nordpol ausbildet.

Um ein besonders elastisches und flexibles galvanisches Element gemäß der vorliegenden Erfindung in Folienform zu schaffen, können die Elektroden 1 und 2 auch dadurch vorgesehen sein, daß entsprechende Metallpulver der entsprechenden Seite der Zwischenschicht 3 im Oberflächenbereich bzw. bis zu einer gewissen Tiefe eingebunden wird. Dadurch ist sichergestellt, daß bei zu starker Biegung oder Knicken der Folie die Elektrode nicht bricht oder reißt, was bei Elektroden möglicherweise der Fall sein könnte, die aus einer Metallschicht bzw. einer Metallplatine bestehen.

Für einige Anwendungsformen kann es auch vorteilhaft sein, wenigstens zwei Lagen des galvanischen Elements übereinander vorzusehen, um den therapeutischen und prophylaktischen Effekt erforderlichenfalls noch zu vergrößern.

Die Abmessungen des erfindungsgemäßen, galvanischen Elements sind in ihrer Länge, Breite und Dicke je nach dem Anwendungsfall beliebig zu wählten. Sie können am Körper direkt angebracht werden.

Als Elektrolyt-Träger hat sich Graphit als besonders vorteilhaft erwiesen. Das Graphit ist bevorzugt porig und der Elektrolyt, beispielsweise Kalilauge, befindet sich in den Poren.

Besonders vorteilhaft ist es, die erfindungsgemäßen galvanischen Elemente in Langzeitanwendung zu verwenden. Dabei wurde festgestellt, daß von diesem erfindungsgemäßen Element insbesondere auch sehr positive Wirkungen hinsichtlich der Schlafeigenschaften bei Menschen ausgehen. Die Verwendung derartiger Elemente im Schlaf- und Bettenbereich ist daher von besonderer Bedeutung. Die erfindungsgemäßen Elemente werden daher vorzugsweise in Folienform, insbesondere an Kissen, Decken, Unterbetten, Oberbetten, Matratzenschoner usw. mit guten Erfolg angebracht.

Die vorliegende Erfindung wurde anhand von Ausführungsbeispielen beschrieben. Dem Fachmann sind jedoch zahlreiche Ausgestaltungen und Abwandlungen möglich, ohne daß dadurch der Erfindungsgedanke verlassen wird. Insbesondere werden sämtliche in den Unterlagen offenbarte Merkmale als erfindungswesentlich beansprucht, soweit sie einzeln oder in Kombination gegenüber dem Stand der Technik neu sind.

**Patentansprüche**

1. Galvanisches Element mit zwei metallelektroden (1, 2) in dem magnetisierbare Teilchen (6) enthalten sind, dadurch gekennzeichnet, daß die magnetisierbaren Teilchen so ausgerichtet sind, daß sich auf der einen metallelektrode der magnetische Süd- und auf der anderen metallelektrode der magnetische Nordpol befindet.

2. Element nach Anspruch 1, dadurch gekennzeichnet, daß das galvanische Element aus zwei Metallelektroden (1, 2) und einer zwischen diesen angeordneten Zwischenschicht (3) besteht, in der ein in Graphit (5) gebundener Elektrolyt vorgesehen ist.

3. Element nach Anspruch 1 oder 2, dadurch gekennzeichnet, daß die Zwischenschicht (3) ein Material auf Kautschukbasis ist, in das Graphit (5) eingelagert ist.

4. Element nach einem der Ansprüche 1 bis 3 dadurch gekennzeichnet, daß in der Zwischenschicht (3) magnetisierbare Teilchen (6) eingelagert sind.

5. Element nach einem der Ansprüche 1 bis 4, dadurch gekennzeichnet, daß Strontiumoxid als magnetisierbare Teilchen (6) eingelagert ist.

6. Element nach einem der Ansprüche 1 bis 4, dadurch gekennzeichnet, daß Eisenoxid als magnetisierbare Teilchen (6) eingelagert ist.

7. Element nach einem der Ansprüche 1 bis 4, dadurch gekennzeichnet, daß eine Mischung aus Strontium- und Eisenoxid als magnetisierbare Teilchen (6) eingelagert ist.

8. Element nach einem der Ansprüche 1 bis 4, dadurch gekennzeichnet, daß anisotropes SrO x 6 Fe$_2$O$_3$ als magnetisierbaret Teilchen (6) eingelagert ist.

9. Element nach einem der Ansprüche 1, 2 und 4 bis 8, dadurch gekennzeichnet, daß in der Zwischenschicht (3) die Elektroden (1, 2) verbindende Bereiche (4) vorgesehen sind, in denen Graphit (5) unregelmäßig eingelagert ist, und die keine magnetisierbaren Teilchen (6) enthalten (Fig. 2).

10. Element nach einem der Ansprüche 1 bis 9, dadurch gekennzeichnet, daß die positive Elektrode (1) aus Kupfer und die negative Elektrode (2) aus Zink besteht.

11. Element nach einem der Ansprüche 1 bis 9, dadurch gekennzeichnet, daß die positive Elektrode (1) aus Gold und die negative Elektrode (2) aus Silber besteht.

12. Element nach einem der Ansprüche 1 bis 11, dadurch gekennzeichnet, daß wenigstens eine Elektrode (1, 2) auf die Zwischenschicht (3) aufgedampft ist.

13. Element nach einem der Ansprüche 1 bis 11, dadurch gekennzeichnet, daß die Elektroden (1, 2) aus Metallpulver bestehen, das auf den jeweiligen Seiten der Zwischenschicht (3) in das Zwischenschichtmaterial eingebracht ist.

14. Element nach einem der Ansprüch 1 bis 13, dadurch gekennzeichnet, daß der Graphit (5) porig ausgebildet ist.

15. Element nach einem der Ansprüche 1 bis 14, dadurch gekennzeichnet, daß der Elektrolyt in den Poren enthalten ist.

16. Element nach einem der Ansprüche 1 bis 15, dadurch gekennzeichnet, daß es in Form einer elastischen Folie ausgebildet ist.

17. Element nach einem der Ansprüche 1 bis 16, dadurch gekennzeichnet, daß es an Bettartikeln angebracht ist.

## Claims

1. Galvanic element having two metal electrodes (1, 2), the element comprising magnetizable particles (6), characterized in that the magnetizable particles are aligned such that the magnetic south-pole being on one metal electrode and the magnetic north-pole being on the other metal electrode.

2. Element according to claim 1, characterized in that the galvanic element comprises two metal electrodes (1, 2) and an intermediate layer (3) between said metal electrodes, the intermediate layer comprises an electrolyte bonded in graphite (5).

3. Element according to claim 1 or 2, characterized in that the intermediate layer (3) is a material on the basis of caoutchouk, the graphite (5) being deposited in said material.

4. Element according to one of the claims 1 to 3, characterized in that magnetizable particles (6) are deposited in the intermediate layer (3).

5. Element according to one of the claims 1 to 4, characterized in that strontium- oxide is deposited as magnetizable particles (6).

6. Element according to one of the claims 1 to 4, characterized in that ferric-oxide is deposited as magnetizable particles (6).

7. Element according to one of the claims 1 to 4, characterized in that a mixture from strontium- and ferric-oxide is deposited as magnetizable particles (6).

8. Element according to one of the claims 1 to 4, characterized in that unisotropic SrO x 6 Fe$_2$O$_3$ is deposited as magnetizable particles (6).

9. Element according to one of the claims 1, 2 and 4 to 8, characterized in that the intermediated layer (3) comprises connecting regions (4) which connect the electrodes (1, 2) in which graphite (5) is irregulary deposited and which do not comprise magnetizable particles (6) (Fig. 2).

10. Element according to one of the claims 1 to 9, characterized in that the positive electrode (1) is composed of copper and the negative electrode (2) is composed of zinc.

11. Element according to one of the claims 1 to 9, characterized in that the positive electrode (1) is composed of gold and the negative electrode (2) is composed of silver.

12. Element according to one of the claims 1 to 11, characterized in that at least one electrode (1, 2) is evaporized on the intermediate layer (3).

13. Element according to one of the claims 1 to 11, characterized in that the electrodes (1, 2) are composed of metalpowder which is placed in the material of the respective side of the intermediate-layer (3).

14. Element according to one of the claims 1 to 13, characterized in that the graphite (5) is porous.

15. Element according to one of the claims 1 to 14, characterized in that the electrolyte is enclosed in the pores.

16. Element according to one of the claims 1 to 15, characterized in that the element is made in form of a elastic.

17. Element according to one of the claims 1 to 16, characterized in that the element is attached to a bed-article.

## Revendications

1. Elément galvanique, comportant deux électrodes métalliques (1, 2) et contenant des particules magnétisables (6), caractérisé en ce que les particules magnétisables sont dirigées de façon que le pôle magnétique Sud se trouve sur une électrode métallique, et le pôle magnétique Nord, sur l'autre.

2. Elément selon la revendication 1, caractérisé en ce que l'élément galvanique est constitué par deux électrodes métalliques (1, 2) et une couche intermédiaire (3) qui est disposée entre elles et dans laquelle il est prévu un électrolyte inclus dans du graphite (5).

3. Elément selon la revendication 1 ou 2, caractérisé en ce que la couche intermédiaire (3) est un matériau à base de caoutchouc dans lequel le graphite (5) est incorporé.

4. Elément selon l'une des revendications 1 à 3, caractérisé en ce que des particules magnétisables (6) sont incorporées dans la couche intermédiaire (3).

5. Elément selon l'une des revendications 1 à 4, caractérisé en ce que de l'oxyde de strontium est incorporé sous forme de particules magnétisables (6).

6. Elément selon l'une des revendications 1 à 4, caractérisé en ce que de l'oxyde de fer est incorporé sous forme de particules magnétisables (6).

7. Elément selon l'une des revendications 1 à 4, caractérisé en ce qu'un mélange d'oxyde de strontium et d'oxyde de fer est incorporé sous forme de particules magnétisables (6).

8. Elément selon l'une des revendications 1 à 4, caractérisé en ce que du $SrO \times 6\ Fe_2O_3$ anisotrope est incorporé sous forme de particules magnétisables (6).

9. Elément selon l'une des revendications 1, 2 et 4 à 8, caractérisé en ce qu'il est prévu, dans la couche intermédiaire (3), des régions (4) qui relient les électrodes (1, 2) et dans lesquelles du graphite (5) est incorporé de façon irrégulière, ces régions ne contenant pas de particules magnétisables (6).

10. Elément selon l'une des revendications 1 à 9, caractérisé en ce que l'électrode positive (1) est en cuivre, et l'électrode négative (2), en zinc.

11. Elément selon l'une des revendications 1 à 9, caractérisé en ce que l'électrode positive (1) est en or, et l'électrode négative (2), en argent.

12. Elément selon l'une des revendications 1 à 11, caractérisé en ce qu'au moins une électrode (1, 2) est obtenue par évaporation sous vide sur la couche intermédiaire (3).

13. Elément selon l'une des revendications 1 à 11, caractérisé en ce que les électrodes (1, 2) sont constituées par de la poudre métallique qui est logée, sur chacune des faces de la couche intermédiaire (3), dans le matériau de la couche intermédiaire.

14. Elément selon l'une des revendications 1 à 13, caractérisé en ce que le graphite (5) est poreux.

15. Elément selon l'une des revendications 1 à 14, caractérisé en ce que l'électrolyte est contenu dans les pores.

16. Elément selon l'une des revendications 1 à 15, caractérisé en ce qu'il est constitué sous la forme d'une feuille élastique.

17. Elément selon l'une des revendications 1 à 16, caractérisé en ce qu'il est monté sur des articles de literie.

# FIG.1

5  6  1

SÜD

POS. ELEKTRODE
( Cu + )

NORD

NEG. ELEKTRODE
( Zn – )

3  2

# FIG.2

6  5  1

SÜD

4  NORD  2  3